Europäisches Patentamt

European Patent Office

Office européen des brevets

(19)

(11) EP 0 989 830 B1

(12) **FASCICULE DE BREVET EUROPEEN**

(45) Date de publication et mention
de la délivrance du brevet:
**21.07.2004 Bulletin 2004/30**

(51) Int Cl.⁷: **A61F 2/01**

(21) Numéro de dépôt: **98924506.3**

(86) Numéro de dépôt international:
**PCT/IB1998/000948**

(22) Date de dépôt: **19.06.1998**

(87) Numéro de publication internationale:
**WO 1998/058599 (30.12.1998 Gazette 1998/52)**

(54) **IMPLANT DE DILATATION INTRAVASCULAIRE A DEFLECTEUR**

INTRAVASKULÄRER STENT MIT EINER ABLENKVORRICHTUNG

IMPLANT WITH DEFLECTOR FOR INTRAVASCULAR DILATION

(84) Etats contractants désignés:
**AT BE CH CY DE DK ES FI FR GB GR IE IT LI LU
MC NL PT SE**
Etats d'extension désignés:
**AL LT LV MK RO SI**

(30) Priorité: **20.06.1997  CH 151497**

(43) Date de publication de la demande:
**05.04.2000  Bulletin 2000/14**

(73) Titulaire: **ECOLE POLYTECHNIQUE FEDERALE
DE LAUSANNE
1015 Lausanne (CH)**

(72) Inventeur: **STERGIOPULOS, Nikolaos
CH-1025 Saint-Sulpice (CH)**

(74) Mandataire: **Micheli & Cie
Rue de Genève 122,
Case Postale 61
1226 Genève-Thonex (CH)**

(56) Documents cités:
**EP-A- 0 433 011        EP-A- 0 722 700
WO-A-93/01764        US-A- 3 868 956
US-A- 5 129 910        US-A- 5 304 194
US-A- 5 573 547**

**Description**

La présente invention concerne un implant intravasculaire permettant la dilatation radiale des parois artérielles. Ces implants ou dilatateurs sont connus sous le nom de 'stent' dans le domaine de l'angioplastie transluminale. L'angioplastie transluminale consiste à traiter des zones malades du système artériel par l'introduction d'appareils, notamment des cathéters par les voies naturelles. Ceci permet des interventions localisées sans devoir recourir à des interventions chirurgicales classiques qui présentent, du fait de leur lourdeur, de nombreux inconvénients pour les patients. Cette technique est notamment utilisée lorsque l'on diagnostique un rétrécissement ou sténose des artères. On introduit alors par l'artère fémorale un cathéter muni à son extrémité distale d'un ballonnet d'angioplastie gonflable. Ce cathéter est ensuite poussé et guidé, sous contrôle radioscopique, à travers le réseau artériel jusqu'à la zone malade de l'artère. Une fois cette zone atteinte, on gonfle le ballonnet pour dilater la zone rétrécie de l'artère. Cette opération est répétée jusqu'à ce que l'on constate grâce aux moyens de contrôle radioscopique, que l'artère présente à nouveau un diamètre suffisant pour assurer un débit sanguin acceptable. Ces interventions présentent toutefois certains inconvénients. En effet, les observations cliniques montrent que dans environ un tiers des cas traités, l'artère se rétrécit à nouveau dans un laps de temps compris entre quelques jours et quelques mois. Ce phénomène que l'on dénomme 'resténose' nécessite une nouvelle intervention sur l'artère malade soit par la même méthode, soit par le biais de techniques chirurgicales plus lourdes.

Pour tenter de résoudre ce problème, on a proposé d'implanter dans l'artère de façon définitive des dilatateurs ou 'stent' pour éviter qu'elle ne se rétrécisse à nouveau. Ces implants présentent usuellement une structure tubulaire ouverte aux extrémités pour ne pas perturber le flux sanguin. Ces dispositifs, indépendamment de leur structure particulière, présentent généralement les caractéristiques suivantes: ils sont extensibles radialement à partir d'un premier diamètre, permettant leur introduction dans l'artère à l'aide d'un cathéter, à un second diamètre plus grand correspondant sensiblement au diamètre de l'artère. Après dilatation de l'artère, ils sont implantés dans cette dernière et prennent appui contre la paroi interne de l'artère empêchant ainsi, par une action mécanique, que l'artère ne se rétrécisse à nouveau. Une fois implantés, ces stent présentent une certaine résistance à la compression radiale et maintiennent ainsi l'artère ouverte tout en permettant l'écoulement du sang. Dans la pratique on utilise couramment des stent de deux types différents. Les premiers sont déformés par le gonflement d'un ballonnet lors de leur mise en place; les seconds stent sont dit auto-extensibles. Les stent auto-extensibles ne nécessitent pas d'action mécanique externe pour passer d'un premier diamètre lors de l'introduction, à un second diamètre plus grand en position de service. Cet effet est obtenu soit par l'utilisation de matériau à mémoire de forme, comme le Nitinol (marque déposée), soit par effet ressort .On a également proposé, par exemple dans le brevet européen EP-433 011 B1, un stent qui comprend un isotope radioactif pour tenter de diminuer le phénomène de resténose par radiothérapie. Dans d'autres réalisations, la surface du stent, en contact avec la paroi interne de l'artère ou du vaisseau, comprend un traitement de surface approprié permettant la distribution locale de substances chimiques anti-thrombogènes.

Ces dispositifs bien qu'ayant contribué à la diminution du taux de resténose, n'ont toutefois pas totalement résolu le problème. On constate en effet, dans environ 22% des cas traités par angioplastie et pose d'un stent, une réaction tissulaire qui conduit à l'augmentation de l'épaisseur de la couche interne de l'artère. Si ce phénomène ne se stabilise pas et continue à croître, l'artère se rebouche.

La présente invention a pour but de remédier aux inconvénients mentionnés ci-dessus en proposant un implant de dilatation favorisant la diminution du taux de resténose, en particulier par son action sur la paroi interne de l'artère. Un autre objet de l'invention consiste dans l'utilisation d'un tel dispositif pour augmenter la contrainte de cisaillement à l'interface sang/paroi dans une artère ou un vaisseau sanguin. Enfin, l'invention a également pour objet un procédé favorisant l'augmentation des contraintes de cisaillement au niveau de la paroi artérielle. Le stent selon la présente invention se distingue à cet effet par les caractéristiques définies à la revendication 1. D'autres avantages ressortent de la description qui suit et des revendications dépendantes.

L'invention va maintenant être décrite en référence aux dessins annexés qui représentent schématiquement et à titre d'exemple non limitatif une forme d'exécution du stent intravasculaire selon l'invention.

La figure 1 est une vue schématique illustrant le profil des vitesses dans une artère sans implant.
La figure 2 est une vue schématique illustrant le profil des vitesses dans une artère comprenant en son centre un déflecteur de flux.
La figure 3 est un graphique illustrant le cisaillement intimal relatif en fonction des dimensions du déflecteur par rapport aux dimensions de l'artère.
La figure 4 est une vue de côté d'un stent selon la présente invention.
La figure 5 est une vue en bout du stent représenté à la figure 4.

Des études scientifiques confirmées par des observations cliniques ont démontré que la resténose est attribuée à une prolifération cellulaire du tissu intimal appelé hyperplasie intimale. Les mécanismes de cette réaction ne sont à ce jour pas encore totalement compris. Cependant, il est certain que la prévention ou l'atténuation de l'hyperplasie intimale constitue un élément clé du succès du traitement des sténoses ou des occlusions des artères. On a constaté chez l'animal que l'hyperplasie intimale est réduite lorsque le débit sanguin est important dans le vaisseau concerné. En revanche, lorsque ce débit est faible la couche intimale augmente. La même constatation a été faite par des cardiologues et des radiologues qui ont observé qu'à la suite d'une angioplastie, les stent restent ouverts si le débit est important et qu'ils ont tendance à se boucher en présence d'un faible débit sanguin. Il existe en conséquence une relation certaine entre le débit sanguin et l'hyperplasie intimale. Ce fait est confirmé par plusieurs études médicales qui tendent à montrer que l'hyperplasie intimale n'est pas un processus pathologique, mais plutôt une réponse adaptative de l'artère ou du vaisseau qui se remodèle de manière à maintenir ou à restaurer un niveau optimal de contrainte de cisaillement à la paroi.

Le passage du sang dans une artère crée par frottement des forces sur la paroi interne de l'artère. Lorsque le débit est important, les forces de cisaillement sont élevées sur les cellules endothéliales de la paroi de l'artère. Ces forces sont au contraire faibles en présence d'un débit insuffisant. On sait par ailleurs que la contrainte de cisaillement à la paroi interne est directement proportionnelle au débit (Q) et inversement proportionnelle au cube du diamètre artériel. Il en découle que lorsque le débit de perfusion est faible, l'hyperplasie intimale réduit le diamètre de l'artère afin de restaurer la valeur normale de la contrainte. Si un faible débit persiste ou s'il diminue progressivement, la contrainte normale de cisaillement ne peut pas être rétablie et l'hyperplasie intimale continue, conduisant finalement à la resténose. Au contraire, si le débit est suffisant pour rétablir un niveau de contrainte égal, voire supérieur, à la contrainte normale, l'hyperplasie intimale s'arrête et l'artère reste ouverte durablement.

Il ressort des constatations énoncées ci-dessus que pour stopper et enrayer l'hyperplasie intimale, il est nécessaire d'augmenter localement la contrainte de cisaillement à la paroi, particulièrement lorsque que le débit est faible. L'objet de l'invention est précisément de permettre une augmentation locale significative de la contrainte de cisaillement à la paroi.

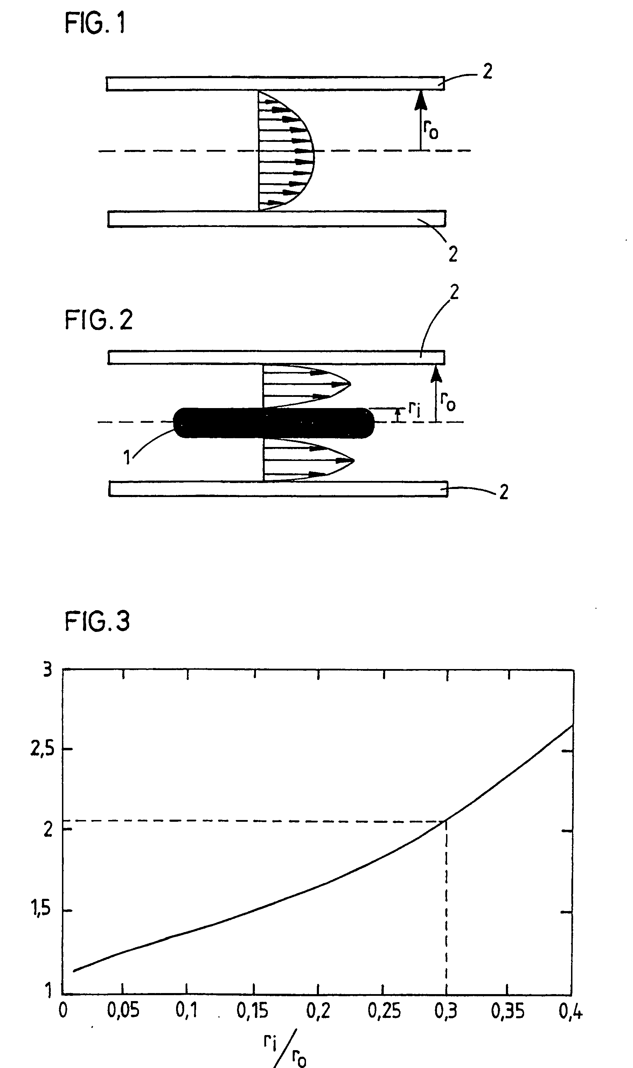

Sachant que le débit sanguin ne peut être augmenté localement, puisqu'il est réglé automatiquement par l'organisme au travers des résistances que constituent les vaisseaux périphériques, il convient en conséquence de diminuer localement la section ouverte de l'artère de telle façon que la valeur de la contrainte de cisaillement sur la paroi de l'artère augmente. A cet effet il est proposé de placer et de maintenir en position, de préférence au centre de l'artère, au moins un corps qui va agir comme un déflecteur du flux sanguin sur la paroi artérielle. Ce déflecteur de flux va ainsi permettre d'augmenter localement de façon significative les contraintes de cisaillement sur les cellules endothéliales. La figure 1 illustre schématiquement le profil des vitesses dans une artère de rayon $r_o$. La figure 2 illustre le même profil des vitesses lorsqu'un déflecteur de flux 1 de forme cylindrique est disposé au centre de l'artère. Le déflecteur 1 dévie les lignes de courant dans la direction radiale en direction des parois artérielles 2 et conduit à un gradient radial de vitesse plus important au voisinage des parois 2 de l'artère. De ce fait, la contrainte de cisaillement à l'interface sang / paroi est augmentée. En référence à la figure 2, et en faisant l'hypothèse que l'écoulement est développé, l'équation de Navier-Stokes selon l'axe de symétrie longitudinal donne :

$$\frac{1}{r} \cdot \frac{\partial}{\partial r} \cdot (r \frac{\partial u}{\partial r}) = -\frac{1}{\mu} \frac{\partial P}{\partial x} \text{ où} \qquad\qquad (1)$$

u est la vitesse axiale,
P la pression et
μ la viscosité du sang.
Par une double intégration, on obtient

$$u(r) = \frac{1}{4\mu} \frac{\partial P}{\partial x} r^2 + c_1 \ln(r) + c_2 \qquad\qquad (2)$$

En appliquant les conditions de bord $u(r = r_i) = u(r = r_0) = 0$ puis en dérivant, on obtient l'expression finale pour la distribution des vitesses

$$u(r) = \frac{1}{4\mu} \frac{\partial P}{\partial x} \left[ r^2 - r_o^2 + \frac{r_i^2 - r_o^2}{\ln\left(\frac{r_o}{r_i}\right)} \ln\left(\frac{r}{r_i}\right) \right] \qquad\qquad (3)$$

Le débit Q peut ensuite être calculé par simple intégration

$$Q = \int_{r_i}^{r_o} u(r)(2\pi r)dr = -\frac{\pi}{8\mu}\frac{\partial P}{\partial x}\left[r_o^4 - r_i^4 - \frac{\left(r_o^2 - r_i^2\right)^2}{\ln\left(\frac{r_o}{r_i}\right)}\right] \tag{4}$$

Les contraintes de cisaillement agissant sur la paroi artérielle $\tau$ sont données par

$$\tau = -\mu\frac{\partial u}{\partial r}\bigg|_{r=r_o} \quad \text{qui en utilisant l'équation 3 pour u(r) devient} \tag{5}$$

$$\tau = -\frac{1}{4}\frac{\partial P}{\partial x}\left[2r_o + \frac{1}{r_o}\frac{r_i^2 - r_o^2}{\ln\left(\frac{r_o}{r_i}\right)}\right] \tag{6}$$

L'équation 6 peut également être exprimée en fonction du débit Q en utilisant l'équation 4 pour le gradient de pression

$$\tau = \frac{2\mu}{\pi}\frac{Q}{r_o^4 - r_i^4 - \frac{\left(r_o^2 - r_i^2\right)^2}{\ln\left(\frac{r_o}{r_i}\right)}}\left[2r_o + \frac{1}{r_o}\frac{r_i^2 - r_o^2}{\ln\left(\frac{r_o}{r_i}\right)}\right] \tag{7}$$

Pour mieux apprécier l'effet du déflecteur 1 sur la grandeur des cisaillements, on normalise cette dernière par les contraintes de cisaillement sous un écoulement laminaire de type Poiseuille de même débit que dans une artère ouverte. Pour un écoulement de type Poiseuille, on sait que :

$$\tau_{Pois} = \frac{4\mu}{\pi r_o^3}\, Q \tag{8}$$

On obtient alors l'expression suivante :

$$\frac{\tau}{\tau_{Pois}} = \frac{1}{2}\frac{r_o^3}{r_o^4 - r_i^4 - \frac{\left(r_o^2 - r_i^2\right)^2}{\ln\left(\frac{r_o}{r_i}\right)}}\left[2r_o + \frac{1}{r_o}\frac{r_i^2 - r_0^2}{\ln\left(\frac{r_o}{r_i}\right)}\right] \tag{9}$$

Il est ainsi possible de définir le rapport des rayons déflecteur/artère comme un paramètre $\gamma = \dfrac{r_i}{r_o}$, pour re-formuler l'équation 9 sous une forme non dimensionnelle :

$$\frac{\tau}{\tau_{Pois}} = \frac{1 + \dfrac{\gamma^2 - 1}{2\ln(\gamma)}}{1 - \gamma^4 - \dfrac{\left(1 - \gamma^2\right)^2}{\ln\left(\dfrac{1}{\gamma}\right)}} \qquad (10)$$

La dépendance des cisaillements par rapport au paramètre y est représentée à la figure 3 sur laquelle on exprime en ordonnée le cisaillement intimal relatif et en abscisse, le rapport entre le rayon du déflecteur et le rayon de l'artère. En prenant par exemple un déflecteur cylindrique dont le rayon correspond environ au tiers du rayon de l'artère, on augmente le cisaillement intimal à la paroi d'un facteur 2. Si comme dans l'exemple mentionné ci-dessus le rapport entre le rayon du déflecteur et celui de l'artère est d'un tiers, la surface occupée par le déflecteur ne représente qu'environ 11% de la section de l'artère et ne constitue de ce fait qu'une résistance négligeable à l'écoulement sanguin selon la mécanique des fluides.

Grâce à la présence d'un corps cylindrique au centre de l'artère provoquant une déviation du flux sanguin, on augmente localement de façon significative la contrainte de cisaillement à la paroi. Ce corps, en raison de ses dimensions, ne diminue pas de façon importante le débit sanguin.

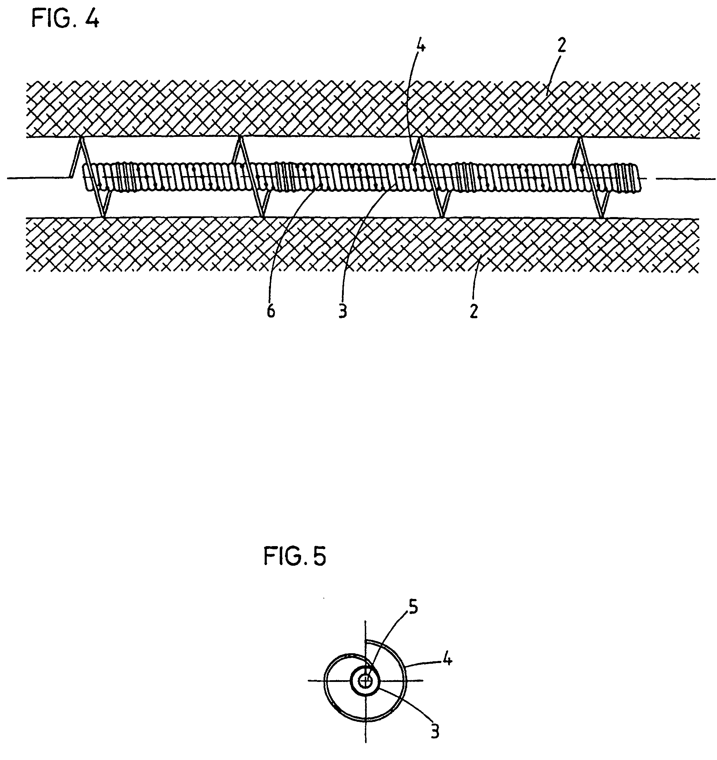

La figure 4 représente une forme d'exécution possible parmi de nombreuses variantes d'un stent selon la présente invention. Ce stent est en place dans une artère ou un vaisseau dont on a représenté schématiquement les parois 2. Il comporte une partie centrale 3 qui remplit la fonction de déflecteur de flux. Ce déflecteur 3 est réalisé à l'aide d'un ressort roulé spire à spire, dans lequel chaque spire est reliée à la spire adjacente par exemple à l'aide d'une soudure au laser. Les points de soudure 6 sont répartis sur une spirale courant sur toute la longueur du ressort. De cette façon, le déflecteur ne peut se déformer selon l'axe longitudinal mais garde néanmoins une certaine souplesse ce qui facilite son acheminement vers la zone à traiter. Aux deux extrémités du déflecteur 3, ainsi qu'à un ou plusieurs emplacements répartis sur sa longueur, de petites spires 4 sont soudées au déflecteur central 3. Ces spires 4 sont extensibles radialement depuis un premier diamètre correspondant approximativement au diamètre du déflecteur 3 vers un second diamètre plus grand correspondant au diamètre de l'artère. Les spires 4 viennent prendre appui, en position de service, sur les parois internes 2 de l'artère et ont la même action mécanique sur la paroi que les stent autoextensibles conventionnels. Ces spires 4 , une fois en contact avec la paroi artérielle, maintiennent le déflecteur 3 en position au centre de l'artère et évitent que ce dernier n'entre en contact avec la paroi annulaire de l'artère. En référence à la figure 5, on voit un passage 5 au centre du déflecteur 3. Ce passage longitudinal 5, qui s'étend sur toute la longueur du déflecteur 3, permet de monter le stent à l'extrémité d'un cathéter d'angioplastie sur un guide à fil pour faciliter sa mise en place dans le vaisseau traité. Pour la réalisation des spires 4, on utilisera de préférence un matériau qui peut être précontraint à une certaine température et qui retrouve sa forme originale à une température plus élevée. Des alliages à base de nickel et de titane tels que le Nitinol (marque déposée) sont parfaitement adaptés pour la réalisation des spires 4. Ainsi, lors de la fabrication du stent, les spires 4 sont refroidies et deviennent donc très malléables. Elles sont alors enroulées autour du déflecteur 3. Le stent est ensuite conditionné dans un cathéter. Lors de la mise en place du stent, après désolidarisation du cathéter et du stent, les spires 4 se réchauffent au contact du sang et se déploient radialement pour venir en contact avec la paroi du vaisseau sanguin. Il va de soi que d'autres techniques connues dans le domaine des stent auto-extensibles peuvent être utilisées. Le déflecteur central 3 peut aussi se présenter sous la forme d'un corps cylindrique plein muni d'un alésage longitudinal central, ou être constitué d'un corps cylindrique creux qui peut le cas échéant servir de réservoir pour une substance à administrer in situ. D'autres variantes de réalisation du déflecteur 3 sont possible, en particulier l'utilisation de plusieurs éléments assemblés comme un double ressort par exemple. Il est également possible de prévoir plusieurs déflecteurs de flux 3 de plus petit diamètre et reliés entre eux, par exemple trois déflecteurs agencés sur les sommets d'un triangle isocèle. Pour ne pas perturber le débit sanguin dans le vaisseau ou l'artère, on choisira un rapport entre le rayon du déflecteur 3 et celui de l'artère se situant entre 0.1 et 0.8, de préférence 0.3. Pour réaliser le stent selon la présente invention, on utilisera de préférence des matériaux biologiquement compatibles tel que le Nitinol (marque déposée) ou l'acier inoxydable. On notera que certains alliages de cuivre peuvent également être envisagés moyennant un traitement de surface adapté, par exemple un revêtement en polyester ou en TEFLON (marque déposée).

Pour minimiser le phénomène de l'hyperplasie intimale, comme cela a été mentionné dans la partie introductive de la description, on a envisagé une action thérapeutique locale soit par un traitement de surface permettant la distribution locale d'une substance anti-resténose, soit par radiothérapie. Ces techniques peuvent facilement être appliquées au stent objet de la présente invention. Il suffit en effet de prévoir un traitement de surface adapté des spires 4 en contact avec la paroi artérielle. L'effet cumulatif d'une augmentation des contraintes de cisaillement au niveau de la paroi est ainsi combiné avec une action radio thérapeutique ou chimique. On notera que non seulement les parties directement en contact avec la paroi artérielle peuvent présenter un traitement de surface adapté, mais également le déflecteur 3.

Il est évident que le stent selon la présente invention peut prendre d'autres formes, la caractéristique essentielle résidant dans la présence d'un déflecteur de flux augmentant la contrainte de cisaillement sur la paroi interne de l'artère et maintenu en position dans l'artère, de préférence au centre de cette dernière. En particulier le stent pourra se présenter sous la forme d'un corps tubulaire ouvert à ses deux extrémités et comportant en son centre un corps cylindrique relié de manière souple au corps tubulaire externe.

Dans certains cas, on ne désire pas laisser le stent de façon définitive dans l'artère. A cet effet certains stent sont réalisés en des matériaux bio-dégradables. Ces matériaux peuvent bien entendu être utilisés pour réaliser un stent selon la présente invention.

Le procédé qui permet d'augmenter localement la contrainte de cisaillement à la paroi d'un vaisseau ou d'une artère comprend les étapes suivantes. On introduit à l'aide d'un cathéter et d'un guide à fil un stent intravasculaire du type décrit ci-dessus jusqu'à la zone malade de l'artère à traiter. Lors de l'acheminement du stent par le réseau artériel, ce dernier présente un diamètre approximativement identique à celui du cathéter. On procède ensuite à la mise en place du stent en libérant ce dernier du cathéter; durant cette opération les spires 4 du stent s'écartent radialement et prennent appui contre la paroi interne de l'artère. Enfin on retire le cathéter, puis le guide à fil.

On remarquera encore que le stent objet de la présente invention est facile à fabriquer et peut être conditionné dans un cathéter, de sorte qu'il est directement utilisable par le praticien.

## Revendications

1. Stent destiné à être introduit dans une artère ou un vaisseau sanguin, **caractérisé en ce qu'**il comporte au moins un déflecteur de flux (3) permettant de dévier les lignes de courant radialement en direction des parois artérielles, induisant ainsi un gradient radial de vitesse plus important au voisinage des parois artérielles (2) ce qui augmente localement la contrainte de cisaillement sur la paroi interne de l'artère (2) ; et **en ce que** le déflecteur de flux (3) est muni de moyens de maintien (4) prenant appui en position de service contre la surface interne du vaisseau (2), lesdits moyens (4) empêchant le déflecteur (3) de venir en contact avec la paroi interne du vaisseau (2) et ayant une action mécanique de maintien de la paroi artérielle.

2. Stent selon la revendication 1, **caractérisé en ce que** le rapport entre le rayon du déflecteur et le rayon de l'artère est compris entre 0.1 et 0.8, de préférence 0.3.

3. Stent selon l'une des revendications précédentes, **caractérisé en ce que** le ou les déflecteurs de flux (3) présentent une forme généralement cylindrique.

4. Stent selon l'une des revendications précédentes, **caractérisé en ce que** le déflecteur (3) est constitué d'un ressort roulé spire à spire où chaque spire est liée à la suivante par un point de soudure (6) et **en ce que** les moyens de maintien sont constitués d'au moins deux spires (4) souples extensibles radialement et reliées au déflecteur (3) par l'une de leurs extrémités.

5. Stent selon la revendication 4, **caractérisé en ce que** les points de soudures (6) sont agencés selon une spirale courant sur toute la longueur du déflecteur (3).

6. Stent selon l'une des revendications précédentes, **caractérisé en ce qu'**il comporte une pluralité de spires (4) extensibles radialement, reliées au déflecteur (3) et réparties à intervalle régulier sur l'axe longitudinal du déflecteur (3).

7. Stent selon l'une des revendications précédentes, **caractérisé en ce qu'**il est réalisé en un matériau à mémoire.

8. Stent selon l'une des revendications précédentes, **caractérisé en ce qu'**il comprend un isotope radioactif efficace pour réduire l'hyperplasie intimale de la paroi artérielle.

9. Stent selon l'une des revendications 1 à 6, **caractérisé par le fait que** les moyens de maintien (4) extensibles radialement et/ou le déflecteur 3 présentent un traitement de surface spécifique ou sont constitués d'un biomatériau permettant la diffusion d'une substance ayant une action sur la paroi artérielle.

10. Stent selon l'une des revendications précédentes, **caractérisé en ce que** le déflecteur (3) comporte un passage (5) central s'étendant sur toute sa longueur.

11. Stent selon l'une des revendications précédentes, **caractérisé en ce qu'**il est réalisé en un matériau bio-dégradable.

## Patentansprüche

1. Stent, dazu bestimmt, in eine Arterie oder ein Blutgefäss eingeführt zu werden, **dadurch gekennzeichnet, dass** er zumindest eine Strömungsablenkvorrichtung (3) umfasst, die es gestattet, die Strömungslinien radial in Richtung auf die Arterienwandungen abzulenken und so in der Nähe der Arterienwandungen (2) einen grösseren radialen Geschwindigkeitsgradienten zu induzieren, was lokal die Scherbeanspruchung der Arterieninnenwandung (2) erhöht; und dadurch, dass diese Strömungsablenkvorrichtung (3) mit Haltemitteln (4) versehen ist, die in der Arbeitsstellung auf der Innenseite des Gefässes (2) ruhen, wobei diese Mittel (4) die Ablenkvorrichtung (3) daran hindern, mit der Gefässinnenwand (2) in Berührung zu kommen, und eine mechanische Haltewirkung auf die Arterienwandung ausüben.

2. Stent nach Anspruch 1, **dadurch gekennzeichnet, dass** das Verhältnis zwischen dem Radius der Ablenkvorrichtung und dem Radius der Arterie zwischen 0,1 und 0,8 und bevorzugt bei 0,3 liegt.

3. Stent nach einem der vorangehenden Ansprüche, **dadurch gekennzeichnet, dass** die Strömungsablenkvorrichtung(en) (3) eine allgemein zylindrische Gestalt aufweisen.

4. Stent nach einem der vorangehenden Ansprüche, **dadurch gekennzeichnet, dass** die Ablenkvorrichtung (3) aus einer Windung an Windung gewickelten Feder besteht, wo jede Windung mit der folgenden durch einen Schweisspunkt (6) verbunden ist, und dadurch, dass die Haltemittel aus zumindest zwei nachgiebigen, radial ausstreckbaren Windungen (4) bestehen, die mit einem ihrer Enden mit der Ablenkvorrichtung (3) verbunden sind.

5. Stent nach Anspruch 4, **dadurch gekennzeichnet, dass** die Schweisspunkte (6) einer Spirale entlang angeordnet sind, die sich über die ganze Länge der Ablenkvorrichtung (3) erstreckt.

6. Stent nach einem der vorangehenden Ansprüche, **dadurch gekennzeichnet, dass** er eine Mehrzahl von radial ausstreckbaren Windungen (4) umfasst, die mit der Ablenkvorrichtung (3) verbunden und in regelmässigen Abständen über die Längsachse der Ablenkvorrichtung (3) verteilt sind.

7. Stent nach einem der vorangehenden Ansprüche, **dadurch gekennzeichnet, dass** er aus einem Gedächtnismaterial gefertigt ist.

8. Stent nach einem der vorangehenden Ansprüche, **dadurch gekennzeichnet, dass** er ein Radioisotop enthält, das wirksam ist, um die intimale Hyperplasie der Arterienwandung zu verringern.

9. Stent nach einem der Ansprüche 1 bis 6, **dadurch gekennzeichnet, dass** die radial ausstreckbaren Haltemittel (4) und/oder die Ablenkvorrichtung (3) eine spezielle Oberflächenbehandlung aufweisen oder aus einem Biomaterial bestehen, wodurch die Diffusion einer Substanz ermöglicht wird, die eine Wirkung auf die Arterienwandung besitzt.

10. Stent nach einem der vorangehenden Ansprüche, **dadurch gekennzeichnet, dass** die Ablenkvorrichtung (3) einen zentralen Durchlass (5) umfasst, der sich über ihre gesamte Länge erstreckt.

11. Stent nach einem der vorangehenden Ansprüche, **dadurch gekennzeichnet, dass** er aus einem biologisch abbaubaren Material gefertigt ist.

**Claims**

1. Stent adapted to be introduced into an artery or a blood vessel, **characterized in that** it comprises at least one flow deflector (3) permitting deflecting the lines of current radially in the direction of the arterial walls, thereby giving rise to a greater radial speed gradient adjacent the arterial walls (2) which locally increases the shear stress on the internal wall of the artery (2); and **in that** the flow deflector (3) is provided with holding means (4) bearing in the service position against the internal wall of the vessel (2), said means (4) preventing the deflector (3) from coming into contact with the internal wall of the vessel (2) and having a mechanical action to support the arterial wall.

2. Stent according to claim 1, **characterized in that** the ratio between the radius of the deflector and the radius of the artery is comprised between 0.1 and 0.8, preferably 0.3.

3. Stent according to one of the preceding claims, **characterized in that** the flow deflector (3) or deflectors have a generally cylindrical shape.

4. Stent according to one of the preceding claims, **characterized in that** the deflector (3) is constituted by a spring rolled turn by turn in which each turn is connected to the following by a solder point (6) and **in that** the holding means are constituted by at least two flexible radially extensible spirals (4) connected to the deflector (3) at one of their ends.

5. Stent according to claim 4, **characterized in that** the solder points (6) are arranged in a spiral extending over all the length of the deflector (3).

6. Stent according to one of the preceding claims, **characterized in that** it comprises a plurality of radially extensible spirals (4) connected to the deflector (3) and distributed at regular intervals along the longitudinal axis of the deflector (3).

7. Stent according to one of the preceding claims, **characterized in that** it is made of a material having shape memory.

8. Stent according to one of the preceding claims, **characterized in that** it comprises a radioactive isotope effective to reduce intimal hyperplasia of the arterial wall.

9. Stent according to one of claims 1 to 6, **characterized by** the fact that the radially extensible holding means (4) and/or the deflector 3 have a specific surface treatment or are constituted of a biologically active material permitting the diffusion of a substance having an action on the arterial wall.

10. Stent according to one of the preceding claims, **characterized in that** the deflector (3) has a central passage (5) extending over all its length.

11. Stent according to one of the preceding claims, **characterized in that** it is made of a biodegradable material.

FIG. 1

FIG. 2

FIG. 3

$r_i/r_o$

FIG. 4

FIG. 5